# EUROPEAN PATENT SPECIFICATION

(11) **EP 1 976 469 B1**
(45) Date of publication and mention of the grant of the patent: **02.06.2010**
(21) Application number: 06809150.3
(22) Date of filing: 30.10.2006
(51) Int. Cl.: A61F 9/01

(54) **DEVICE AND METHOD FOR CALIBRATING A LASER SYSTEM**
VORRICHTUNG UND VERFAHREN ZUM KALIBRIEREN EINES LASERSYSTEMS
DISPOSITIF ET PROCEDE DE CALIBRAGE D'UN SYSTEME A FAISCEAU LASER

(30) Priority: 25.01.2006 US 339309
(43) Date of publication of application: 08.10.2008
(73) Proprietor: Technolas Perfect Vision GmbH, 80992 München (DE)
(72) Inventor: KESSLER, Ralf, D-69120 Heidelberg (DE); MARTIN, Marcel, D-85748 Garching (DE); WÜHL, Stefan, 69115 Heidelberg (DE); KUHN, Tobias, 07745 Jena (DE); VON PAPE, Ulrich, 67346 Speyer (DE)
(74) Representative: Ullrich & Naumann
(86) International application number: PCT/IB2006/003057
(87) International publication number: WO 2007/085891

(56) References cited:
- EP-A- 1 169 985
- EP-A- 1 364 632
- WO-A-99/04220
- US-A1- 2002 026 180

## Description

### FIELD OF THE INVENTION

The present invention pertains generally to laser system calibration procedures. More particularly, the present invention relates to systems and methods for performing laser system calibration wherein the laser beam causes Laser Induced Optical Breakdown (LIOB) in a reference material. The present invention is particularly, but not exclusively, useful as a system and method for precise calibration of a laser system via the identification and measurement of locations where LIOB occurs in a reference material.

### BACKGROUND OF THE INVENTION

For a laser system used in ophthalmic surgery, it is critical that the laser beam be properly focused, and that the position of the beam's focal point with respect to the laser generating unit be known. Further, due to the curved nature of the cornea, a beam that is to be used in ophthalmic surgery must exhibit proper depth and avoid tilt and lateral displacement (decentration). Additionally, the focal point of the laser beam should have a substantially constant energy density at all positions of the treatment area. Proper calibration of laser systems in this field requires the collective consideration of all these factors (i.e. focal point position, energy density, and overall beam orientation). This is particularly important because an inaccurately or improperly directed laser beam could cause permanent damage to an area of the eye not intended for treatment.

While properly calibrated laser systems are vital to improving the results of ophthalmic surgery, it has heretofore proven difficult to properly calibrate laser systems to the high level of precision desired. In light of the above, it is an object of the present invention to provide an efficient device and method for calibrating a surgical laser system. Another object of the present invention is to provide a device and method in which a lateral displacement of the beam is translated to a z-axis displacement in a calibration member. Another object of the invention is provide a device and method for identifying the position of the focal point in the z-axis. It is yet another object of the present invention to provide a laser calibrating device and method that allows for identification of tilt and decentration of the laser beam. Still another object of the present invention is to provide a device and method for calibrating a laser system that is easy to perform and is comparatively cost effective.

EP-A2-1169985 is considered to represent the closest prior art and discloses a laser system comprising a ultra-short pulse laser unit for generating a laser beam, optical means for focusing the laser beam to a focal point at a preselected initial point, and means for moving the focal point in an axial direction. Since the device of D1 is intended for use in intrastromal eye surgery, precise placement (in three dimensions) of the actual focal point is essential, and as a result the system will invariably have been calibrated in terms of the positioning of the focal point, but this document is silent in particular as to the calibration visualisation means. WO-A-9904220 discloses the use of ablatable plastic blanks for the purpose of calibrating a surgical laser.

### SUMMARY OF THE INVENTION

The invention is defined as the system of appended claim 1 and the method of appended claim 1.

The surgical laser system includes a laser unit for generating a laser beam, preferably a femtosecond laser beam. Within the context of the present invention, the laser unit is considered to define a base datum that may be used as a spatial reference for calibration procedures. Further, the system includes a calibration body that is mounted on the laser unit. For the purposes of the present invention, a calibration member that is made of a material having a predetermined energy threshold for LIOB is affixed to the calibration body.

Structurally, the calibration member includes a surface that defines a central axis which is substantially perpendicular thereto. Preferably, the surface of the calibration member has a predetermined curvature with a radius of curvature in a range between about eight and twelve millimeters. When the calibration member is affixed to the calibration body, and the calibration body is mounted on the laser unit, the surface of the calibration member is positioned at a predetermined distance from the base datum of the laser unit. Also, the central axis of the calibration member is substantially aligned with the expected path of the laser beam, and it passes through the apex of the surface of the calibration member.

For the present invention, the system also includes a mechanism for focusing the laser beam to a focal point at a pre-selected initial location, and then moving the laser beam in the z-direction towards an expected final location. In this movement, each location of the focal spot corresponds to a specific configuration C of the focusing mechanism. Thus, the pre-selected initial location for the focal spot will correspond to an initial configuration C₀ of the focusing mechanism. Once C₀ is established, the focal spot is then moved toward the expected final location. Importantly, if the laser beam is properly calibrated, the expected final location of the focal point will be incident on the surface of the calibration member, and the focusing mechanism will have a configuration C_{E}. Otherwise, an early appearance, or a complete absence, of LIOB on the surface indicates the laser unit is out of calibration in a z-direction. With an absence of LIOB, the final location of the focal point (corresponds to C_{E}), needs to be further moved in the z-direction (i.e. along the central axis) until LIOB does, in fact, occur at the surface. Regardless whether LIOB occurs earlier than expected, or after further z-movement, the eventual location where LIOB can be observed on the surface is referred to hereinafter as the actual final location and corresponds to a configuration C_{A} of the focusing mechanism. In this process, if the upper surface of the calibration member is being used for a z-calibration, the focal point is moved toward the calibration member and away from the laser unit. On the other hand, if it is the lower surface of the calibration member that is being used, the focal point is moved back, toward the laser unit. In either case, the distance "d", between the expected final location (corresponding to C_{E}) and the actual final location (corresponding to C_{A}), is determined. Thus, the distance "d" is represented by the difference between the configurations C_{E} and C_{A} of the focusing mechanism. It is this distance "d" that is then used in the calibration of the laser system for its z-location. This, however, does not end the calibration process. Once the actual final location (i.e. z-correction corresponding to C_{A}) has been calibrated for the laser system, it is still necessary to calibrate for tilt and decentration.

Additionally, for all calibration evaluations, the system is provided with an imaging device for identifying whether LIOB is induced. A measurement device is also provided for measuring the distance "d" and a radial distance "r" of the final LIOB locations from the central axis to calibrate the laser beam. In this manner, the control of the laser beam may be calibrated.

During operation of the system, a plurality of final LIOB locations may be used to calibrate a "tilt" and a "decenter" for the laser beam. Further, a plurality of final LIOB locations may be used to create test patterns from laser beams having different energies. Specifically, energies are provided in a range between a low energy and a high energy, yielding an energy density at the focal point below the energy density threshold for LIOB of the calibration member, and a high energy density at the focal point above the energy density threshold for LIOB of the calibration member, to determine an energy density for the focal spot of the laser beam. Further, a plurality of test patterns may be compared with each other to determine a uniformity for energy density in the focal spot of the laser beam.

### BRIEF DESCRIPTION OF THE DRAWINGS

The novel features of this invention, as well as the invention itself, both as to its structure and its operation, will be best understood from the accompanying drawings, taken in conjunction with the accompanying description, in which similar reference characters refer to similar parts, and in which:
Fig. 1 is a cross sectional view of an embodiment of the device for calibrating a laser system of the present invention;
Fig. 2 is a schematic view, not to scale, of the focal point of the laser beam of the system of Fig. 1 being directed into contact with the surface of the calibration member of Fig. 1 in accordance with the present invention;
Figs. 3A, 4A, 5A and 6A are schematic elevation views, not to scale, of the system of Fig. 1 wherein the focal points of the respective laser beams are directed into contact with the calibration member while being directed in a circular path in accordance with the present invention;
Figs. 3B, 4B, 5B, and 6B are respective plan views of the calibration member that correspond to laser beam directions indicated in Figs. 3A, 4A, 5A, and 6A; and
Figs. 7A and 7B are plan views of respective calibration members to which various laser beam focal point patterns have been applied in accordance with the present invention.

### DESCRIPTION OF THE PREFERRED EMBODIMENTS

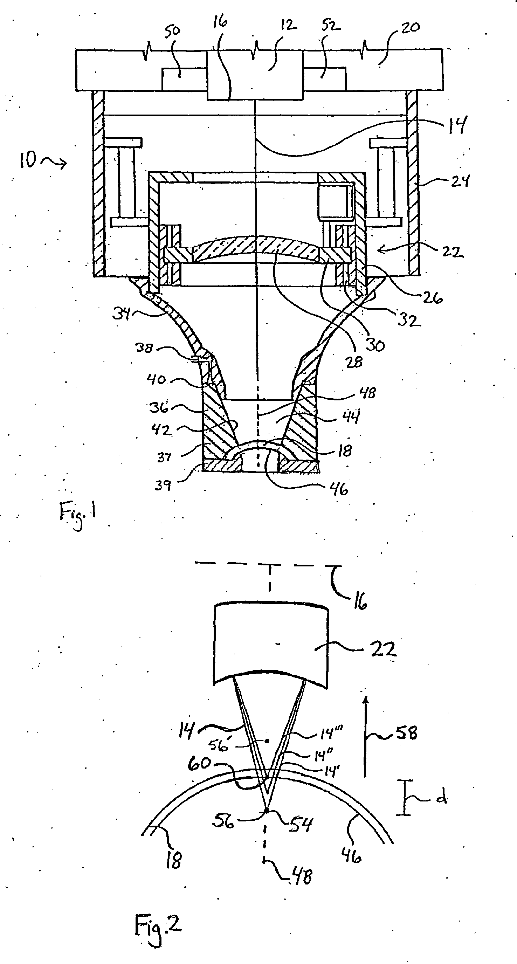

Referring initially to Fig. 1, a system for calibrating a, preferably femtosecond, laser system in accordance with the present invention is shown and generally designated 10. As shown, the system 10 includes a laser unit 12 for generating the laser beam 14. Further, the laser unit 12 defines a base datum 16 that will be used as a spatial reference for the calibration procedures performed by the system 10. As will be explained below, the system 10 relies on a calibration member 18 to calibrate the laser unit 12. However, in order to properly explain the system 10, the general components used to focus the laser beam 14 are first identified and discussed.

Structurally, the laser unit 12 is mounted on a housing 20. The laser unit 12 can be of any type well known in the art which is capable of generating an ophthalmic laser beam 14. Furthermore, while a specific optical arrangement that can be used to direct the laser beam 14 through system 10 is shown, it is to be appreciated that any known optical arrangement can be employed. As shown in Fig. 1, the housing 20 is fixedly attached to a mechanism 22 for focusing the laser beam 14. Specifically, the housing 20 is connected to a substantially cylindrical base 24 of the mechanism 22. Further, the mechanism 22 includes a substantially cylindrical frame 26 to which the base 24 is connected.

Still referring to Fig. 1, the system 10 is shown to have an objective lens 28 for focusing the laser beam 14. Structurally, the lens 28 is held in a bracket 30 which has projections (not shown). Further, the base 24 includes tracks 32 that receive and mate with the projections. As a result of this cooperation of structure, the lens 28 may be moved toward or away from the laser unit 12 to focus the laser beam 14 along a prescribed path for completion of the desired calibrating procedure. Alternatively, the lens 28 may be fixed and the focal point can be moved by changing the divergence of the laser beam 14. It is, of course, within the scope of the present invention to use any other type of mechanism which allows control of the focus of the laser beam 14 relative to the base datum 16. It is further to be appreciated that a specific configuration C of the mechanism corresponds to a specific position of the focal spot.

As shown, the frame 26 is fixed to a substantially cylindrical alignment device 34. Further, during a calibration procedure the alignment device 34 is held against the substantially cylindrical calibration body 36. As shown in Fig. 1, the calibration body 36 preferably includes an upper part 37 and a lower part 39 that can be selectively engaged, or disengaged from each other. Thus, the calibration member 18 can be held between the upper part 37 and the lower part 39 when they are engaged. As envisioned for the present invention, the parts 37 and 39 can be engaged and held together in any manner well known in the pertinent art, such as by screws (not shown). The intent here is that, after a procedure has been completed, the parts 37 and 39 can be disengaged and the calibration member 18 removed for further, more precise, evaluation. A new calibration member 18 can then be incorporated with the calibration body 36 and used for the test and evaluation of another, subsequent calibration procedure. More specifically, the subsequent evaluation can be accomplished using an external microscope with better resolution than could be obtained using only a surgical microscope that may be included as part of the laser unit 12. As also shown in Fig. 1, the alignment device 34 is provided with a channel 38. The channel 38 is positioned adjacent the interface 40 between the upper part 37 of alignment device 34 and the calibration body 36. The channel 38 can be connected to a vacuum pump (not shown) to create a partial vacuum in the channel 38 to hold the alignment device 34 against the calibration body 36 during a calibration procedure.

Still referring to Fig. 1, it can be seen that the cylindrical calibration body 36 has an internal face 42 defining a hole 44 for passage of the laser beam 14. Spanning the hole 44 is the calibration member 18, which is made of a material having a predetermined arid well defined energy threshold for LIOB. The calibration body 36 and member 18 may be unitary or separate components as disclosed above. As shown, the calibration member 18 includes a surface 46 defining a central axis 48 that passes through the apex of the surface 46 and is substantially perpendicular thereto. In one aspect of the present invention, the surface 46 is opposite the laser unit 12 from the calibration member 18. Stated differently, the calibration member 18 is between the laser unit 12 and the surface 46. It is to be appreciated, however, that the surface 46 can also face the laser unit 12. In this case, the surface 46 is between the calibration member 18 and the laser unit 12. In both instances, while the surface 46 is shown to be curved, it may, alternatively, be flat. Typically, the surface 46 has a similar shape to the patient interface (cornea) used during surgery. In certain embodiments, the surface 46 has a predetermined curvature with a radius of curvature in a range between about eight and twelve millimeters. When assembled, the surface 46 is positioned at a predetermined distance from the base datum 16 of the laser unit 12. As a result, the system 10 provides for precise calibration of the laser beam 14 relative to the surface 46 of the calibration member 18.

As further shown, the system 10 is provided with an imaging device 50, such as a Charge-Coupled Device (CCD) camera or a surgical microscope and camera assembly, for identifying whether LIOB has occurred in the calibration member 18. Specifically, the imaging device 50 is shown mounted to the housing 20 adjacent the laser unit 12. Additionally, a measurement device 52 may be mounted to the housing 20 to measure the position of the focal point of the laser beam 14 relative to the base datum 16 (distance) and the central axis 48 (radial distance).

Referring now to Fig. 2, the use of the calibration member 18 to calibrate the z-axis control of the laser beam 14 will be explained. As shown, the laser beam 14 is directed by the focusing mechanism 22 to a focal point 54 at a pre-selected initial location 56. In this initial stage, although the beam energy density at focal point 54 is sufficient to cause LIOB of the material in the calibration member 18, the maximum beam energy density reached in the beam 14' upstream of the focal point 54 is insufficient to cause LIOB in the calibration member 18. Thereafter, the focal point 54 is moved in the direction of arrow 58 coincident with or parallel to the central axis 48 in µm steps from beam 14' to 14" to 14"'. At the end of the movement of the focal point 54 toward the calibration member 18, the focal point 54 of beam 14"' reaches the surface 46 of the calibration member 18 and LIOB occurs at this final location 60. For the present invention, an imaging device 50 (shown in Fig. 1) identifies the plasma spark associated with the occurrence of LIOB in the calibration member 18. Alternatively, the occurrence of LIOB may be identified by an operator. For the circumstance wherein the focal point 54 is at an initial location 56' that is above, instead of below, the calibration member 18, a reverse movement of the focal point 54 away from the focusing mechanism 22 and toward the calibration member 18 is required. The consequence is essentially the same.

Upon identification of LIOB, movement of the focal point 54 in the direction of arrow 58 is ceased. Thereafter, the z-position of the final location 60 relative to the base datum 16 may be measured by the measurement device 52 (shown in Fig. 1) or derived from the configuration C of the focusing mechanism 22, and the z-axis control of the laser system 10 can be calibrated.

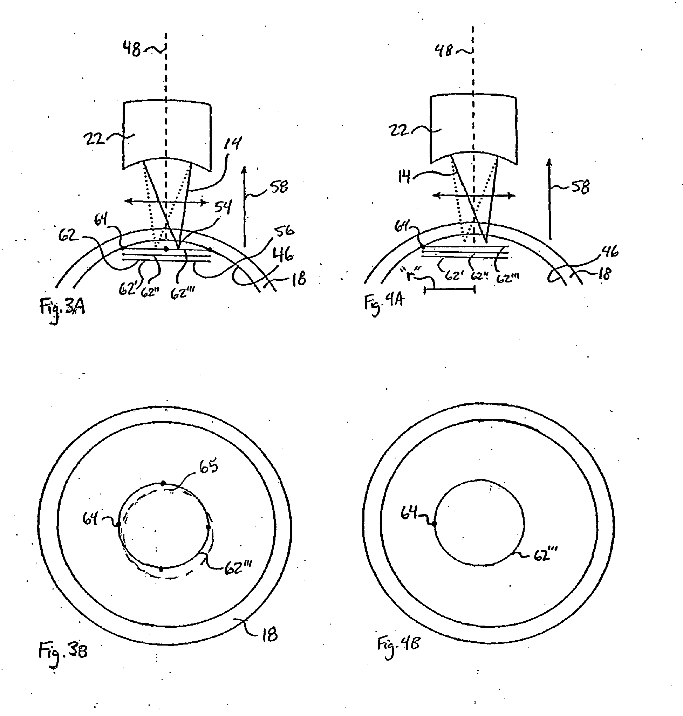

Referring now to Figs. 3A and 3B, the ability to calibrate tilt and decentration of a beam 14 from a laser unit 12 (shown in Fig. 1) is illustrated. As shown, the focal point 54 of the beam 14 is moved on circular paths 62 within the periphery of the surface 46. As described above, the focal point 54 is first positioned at an initial location 56 downstream of the surface 46 and then is moved in the direction of arrow 58 toward the surface 46 preferably in two micron steps. The focal point 54 moves from circular path 62' through path 62" to path 62'" where it contacts the surface 46 of the calibration member 18 at a location 64. For the present invention, while continuing along circular paths 62, the focal point 54 is moved upward until a full path 62"' is completed within the calibration member 18. Then, the plurality of locations 64 is used to calibrate a "tilt" and a "decenter" for the laser beam 14. Specifically, the imaging device 50 (shown in Fig. 1) records both the z-axis position of the first contact between the focal point 54 and the surface 46 as well as the z-axis position of the first circular path 62'" completely within the calibration member 18. Because the laser beam 14 shown in Fig. 3A is in perfect alignment with the calibration surface 46, LIOB will occur substantially simultaneously upon first contact between the focal point 54 and the surface 46 around the circular path 62"'. While only four locations 64 are shown in Figs. 3A and 3B for purposes of clarity, it should be understood that LIOB occurs along the entire circular path 62'" in this example.

A determination as to whether the circular path 62"' actually results from a perfect alignment of the laser beam 14 can be easily verified. Specifically, this can be done after completion of the procedure disclosed immediately above. By rotating the calibration body 36 through a predetermined angle about the central axis 48 (e.g. 90° or 180°), a verification path 65 (shown as a dashed circle in Fig. 3B) can be made into the calibration member 18. When the verification path 65 is congruent with the circular path 62"', a perfect alignment of the laser beam 14 with the calibration member 18 is indicated. On the other hand, if the verification path 65 is displaced relative to the circular path 62"' (as shown in Fig. 3B), further evaluation of tilt and decentration is required.

Referring now to Figs. 4A and 4B, a calibration process is illustrated for a beam 14 that is laterally displaced to the left. As a result of this decentration, the first location 64 of LIOB, due to contact between the focal point 54 and the surface 46, is shifted to the left from the example seen in Figs. 3A and 3B. The radial distance between location 64 and the central axis 48 is represented by "r". Further, LIOB only occurs at the location 64 in the path 62'" as shown in Fig. 4B. As can be understood, there would be a significant difference in the z-axis position of the location 64 and the first circular path (not shown) completely within the calibration member 18 for a beam 14 with decentration. This z-axis difference can be measured and used to calibrate the beam 14 based on the known curvature of the calibration surface 46.

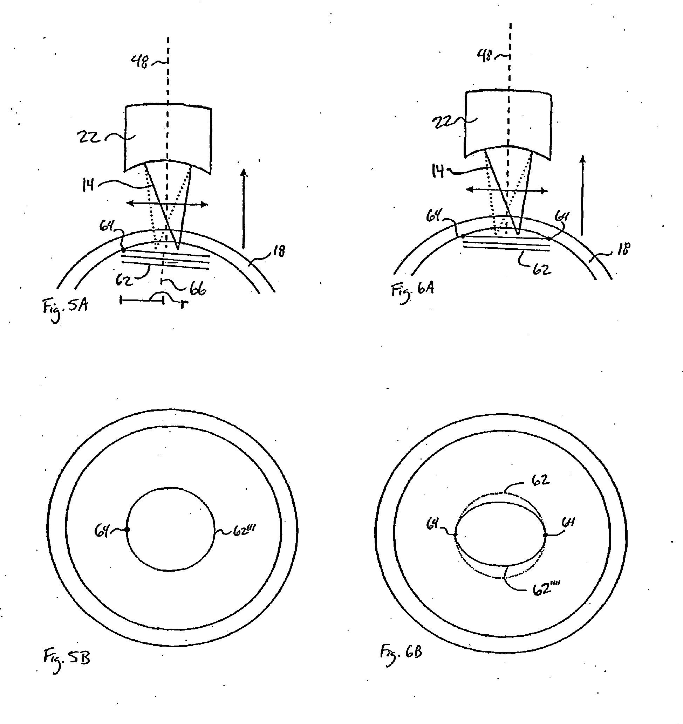

Referring now to Figs. 5A and 5B, a calibration process is illustrated for a beam 14 experiencing tilt. As in Fig. 4A, LIOB is shown first,occurring on the left side at location 64 which is spaced from the central axis 48 by a radial distance "r". The first circular path 62 to be completely within the calibration member 18 will be centered about the optical axis 66 which forms an angle with the central axis 48. As both the initial location 64 of LIOB and the first circular path 62 fully within the member 18 are recorded by the imaging device 50 and measured by the measurement device 52 (both shown in Fig. 1), the beam 14 may be calibrated to correct the tilt.

Referring now to Figs. 6A and 6B, a calibration process is illustrated for a beam 14 having an elliptical path 62"", such as might be caused by a mismatch of the galvanometric scanners (not shown) of the focusing mechanism 22. In this case, LIOB first occurs only at locations 64 along the long axis of the elliptical path 62"". Further, the first path 62 fully within the calibration member 18 will be elliptical. Again, the imaging device 50 and measurement device 52 (both shown in Fig. 1) will identify and measure the locations 64 and the first path 62 fully within the member 18 to calibrate the laser beam 14. For the present invention, if the curvature of the surface 46 is comparable to the curvature of a typical cornea, i.e., around 8-12 mm radius of curvature, and if paths 62 with a radius of about 5 mm are cut, then a lateral displacement of the beam 14 will have approximately the same effect on z-axis displacement, i.e., a lateral displacement of 10 µm will result in a z-axis displacement of about 10 µm. As a result, if the path 62 of the beam 14 is moved in the direction 58 in 2 micron steps, LIOB would occur along the long axis five steps before LIOB occurs along the entire path 62. Therefore, the elliptical nature of the path 62 would be easily identified. On the other hand, due to the typically limited resolution and/or magnification of a standard surgical microscope installed in laser systems 10, it would not be possible to detect a 10 micron difference between the two axes of an ellipse by measuring the ellipse.

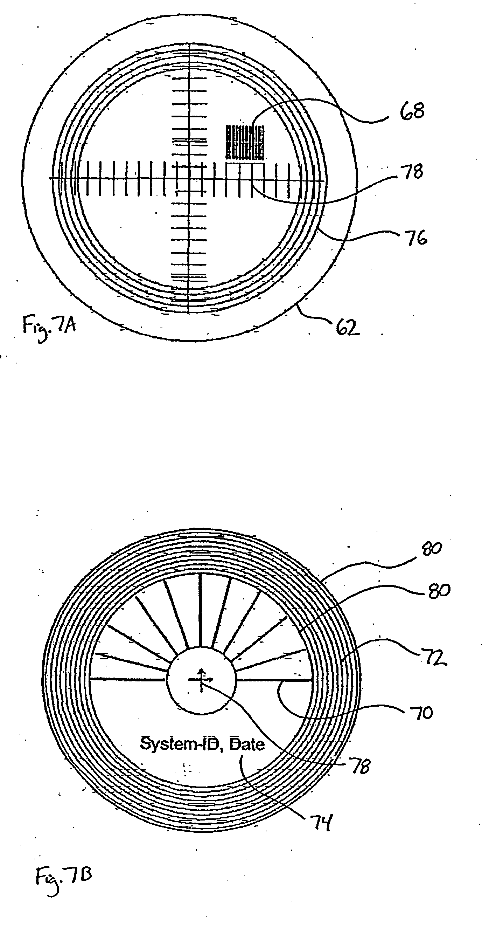

As is understood from Figs. 2-6B, the z-axis position, tilt/decenter, and any ellipticity of laser beam 14 may be determined and calibrated using the system 10. Once the calibration is performed, the laser beam 14 may be used to apply test patterns comprising a plurality of final locations 60 within the calibration member 18. Specifically, the test patterns may be applied via LIOB to check the energy density in the focal spot as well as the uniformity of the energy density over the treatment area. As shown in Figs. 7A and 7B, the energy density within the focal point 54 can be determined by directing the focal point 54 through the calibration member 18 along a path defined by an energy band 68, spokes 70, or circles 72 with increasing energy levels. Preferably each test pattern is respectively created using a different energy in the laser beam 14 in a range between a low energy and a high energy, yielding an energy density at the focal point below the energy density threshold for LIOB of the calibration member, and a high energy density at the focal point above the energy density threshold for LIOB of the calibration member, to determine an energy density within the focal spot of the laser beam 14. Typically, LIOB will occur at a certain energy level, i.e., at a certain position in the energy band 68, at a certain spoke 70, or at a certain circle 72. As discussed above, the occurrence of LIOB can be detected by the operator or by the imaging device 50 (shown in Fig. 1).

Further, a plurality of test patterns may be compared with each other to determine the uniformity of the energy density in the focal spot of the laser beam 14. For instance, the uniformity of the energy density can be determined by looking at circles 72 with different energy levels. If no fluctuations in the energy density in the focal spot of the beam 14 are present, then each circle 72 will have an even intensity. Intensities will vary only between circles 72 formed with different beam energies. If there are fluctuations, then parts of the circles 72 will appear fainter or may disappear. Preferably, the test patterns are created within the material of the calibration member 18. With this in mind, the thickness of the calibration member 18, between its upper and lower surfaces, will typically be about 0.5 millimeters.

As shown in Fig. 7B, the beam 14 can be used to apply system information 74 to the calibration member 18 for archiving purposes. As further seen in Figs. 7A and 7B, circles 76 having different depths, a cross-hair/scale 78, and reference circles 80 having predetermined diameters may be applied to the calibration member 18.

While the particular Device and Method for Calibrating a Laser System as herein shown and disclosed in detail is fully capable of obtaining the objects and providing'the advantages herein before stated, it is to be understood that it is merely illustrative of the presently preferred embodiments of the invention and that no limitations are intended to the details of construction or design herein shown other than as described in the appended claims.

## Claims

1. A calibratable surgical laser system **(10)** adapted for calibration of the position of a focal point **(54)** of the laser system **(10),** which comprises:
a laser unit **(12)** for generating a laser beam **(14),** wherein said laser unit **(12)** defines a base datum **(16);**
a calibration body **(36)** mounted on said laser unit **(12);**
a calibration member **(18)** affixed to said calibration body and having a curved surface **(46)** with a predetermined curvature, wherein the surface **(46)** defines a central axis **(48)** substantially perpendicular thereto, and wherein said calibration member **(18)** is affixed to said calibration body **(36)** to position the surface **(46)** of said calibration member **(18)** at a predetermined distance from the base datum **(16)** of said laser unit **(12);**
an optical means **(22)** for focusing the laser beam **(14)** to a focal point **(54)** at a pre-selected initial location **(56)** at a distance from the surface **(46)** of the calibration member **(18)**;
a means for moving the focal point toward the surface **(46)** of said calibration member **(18)** through a distance "d", until a Laser induced Optical Breakdown (LIOB) is induced at a final location **(60)** on the surface **(46)** of said calibration member **(18)**;
a means for determining the distance "d"; and
a means for comparing the distance "d" with a predetermined value to calibrate the laser system **(10)**.

2. A system **(10)** as recited in claim 1 further comprising a means for measuring a radial distance "r" of the final location **(60)** from the central axis **(48)** to calibrate the laser system **(10).**

3. A system **(10)** as recited in claim 1 , wherein the surface **(46)** has a radius of curvature in a range between about eight and twelve millimeters.

4. A system **(10)** as recited in claim 1 wherein said calibration member **(18)** is made of a material having a predetermined energy threshold for LIOB.

5. A system **(10)** as recited in claim 1 wherein a plurality of final locations **(60)** is used to calibrate a "tilt" and a "decenter" for the laser system (10), wherein preferably a plurality of final locations **(60)** creates a test pattern.

6. A system **(10)** as recited in claim 5 wherein each test pattern is respectively created using a different energy in the laser beam **(14),** and wherein the respective different energies are in a range between a low energy and a high energy, yielding an energy density at the focal point **(54)** below the energy density threshold for LIOB of the calibration member **(18),** and a high energy density at the focal point **(54)** above the energy density threshold for LIOB of said calibration member **(18)** to determine an energy density for the focal spot of the laser beam **(14),** wherein preferably a plurality of test patterns are compared with each other to determine a uniformity for energy density in the focal spot of the laser beam **(14).**

7. A method for calibrating the position of a focal point **(54)** of a surgical laser system **(10),** which comprises the steps of:
supplying a means **(12)** for generating a laser beam **(14)**
wherein said generating means **(12)** defines a base datum **(16);**
mounting a means **(36)** for calibrating the laser beam **(14)** to the generating means **(12),** with said calibrating means **(36)** including a curved calibration member surface **(46)** with a predetermined curvature, wherein the surface **(46)** defines a central axis **(48)** substantially perpendicular thereto, and wherein the surface **(46)** is positioned at a predetermined distance from the base datum **(16)** of said generating means **(12);**
generating the laser beam **(14)** with the generating means **(12)** to pass the laser beam **(14)** through said calibration member surface **(46);**
focusing the laser beam **(14)** to a focal point **(54)** at a preselected initial location **(56)** at a distance from the calibration member surface **(46);**
moving the focal point through a distance "d", until a Laser Induced Optical Breakdown (LIOB) is induced at a final location **(60)** on the surface **(46)** of said calibration member **(18);**
determining the distance "d"; and
comparing the distance "d" with a predetermined value to calibrate the laser system **(10).**

8. A method as recited in claim **7** further comprising the step of measuring a radial distance "r" of the final location **(60)** from the central axis (48) to calibrate the laser system **(10).**

9. A method as recited in claim **7** further comprising the steps of:
directing the focal point **(54)** away from the surface **(46);** and
repeating the moving and directing steps to induce LIOB at a plurality of final locations **(60)** on the surface **(46)** in order to calibrate a "tilt" and a "decenter" for the laser beam **(14).**

10. A method as recited in claim **9** wherein the plurality of final locations **(60)** creates a test pattern, and wherein the moving and directing steps are performed multiple times to create a plurality of test patterns, with each test pattern respectively created using a different energy in the laser beam **(14),** and wherein the respective different energies are in a range between a low energy and a high energy, yielding an energy density at the focal point **(54)** below the energy density threshold for LIOB of the calibration member **(18),** and a high energy density at the focal point **(54)** above the energy density threshold for LIOB of said calibration member **(18)** to determine an energy density for the focal spot of the laser beam **(14),** preferably further comprising the step of comparing the plurality of test patterns with each other to determine a uniformity for energy density in the focal spot of the laser beam **(14).**

## Patentansprüche

1. Kalibrierbares chirurgisches Lasersystem (10), das zur Kalibrierung der Position eines Brennpunkts (54) des Lasersystems (10) angepasst ist und umfasst:
eine Lasereinheit (12) zum Erzeugen eines Laserstrahls (14), wobei die Lasereinheit (12) einen Basispunkt (16) definiert,
einen Kalibrierkörper (36), der an der Lasereinheit (12) befestigt ist,
ein Kalibrierelement (18), das an dem Kalibrierkörper befestigt ist und eine gekrümmte Oberfläche (46) mit einer vorgegebenen Krümmung aufweist, wobei die Oberfläche (46) eine im Wesentlichen senkrecht zu ihr verlaufende zentrale Achse (48) definiert und wobei das Kalibrierelement (18) an dem Kalibrierkörper (36) zum Positionieren der Oberfläche (46) des Kalibrierelements (18) in einem vorgegebenen Abstand von dem Basispunkt (16) der Lasereinheit (12) befestigt ist,
ein optisches Mittel (22) zum Fokussieren des Laserstrahls (14) in einen Brennpunkt (54) an einem vorgewählten anfänglichen Ort (56) in einem Abstand von der Oberfläche (46) des Kalibrierelements (18),
ein Mittel zum Bewegen des Brennpunkts zu der Oberfläche (46) des Kalibrierelements (18) um einen Abstand "d", bis ein Laser Induced Optical Breakdown (LIOB) an einem endgültigen Ort (60) auf der Oberfläche (46) des Kalibrierelements (18) induziert ist,
ein Mittel zum Bestimmen des Abstands "d" und
ein Mittel zum Vergleichen des Abstands "d" mit einem vorbestimmten Wert, um das Lasersystem (10) zu kalibrieren.

2. System (10) nach Anspruch 1, weiter umfassend ein Mittel zum Messen eines radialen Abstands "r" des endgültigen Orts (60) von der zentralen Achse (48), um das Lasersystem (10) zu kalibrieren.

3. System (10) nach Anspruch1, wobei die Oberfläche (46) einen Krümmungsradius in einem Bereich zwischen etwa acht und zwölf Millimetern aufweist.

4. System (10) nach Anspruch 1, wobei das Kalibrierelement (18) aus einem Material hergestellt ist, das eine vorbestimmte Energieschwelle für LIOB aufweist.

5. System (10) nach Anspruch 1, wobei eine Vielzahl von endgültigen Orten (60) verwendet wird, um eine "Neigung" und eine "Dezentrierung" des Lasersystems (10) zu kalibrieren, wobei vorzugsweise eine Vielzahl von endgültigen Orten (60) ein Testmuster erzeugt.

6. System (10) nach Anspruch 5, wobei jedes Testmuster jeweils unter Verwendung einer unterschiedlichen Energie in dem Laserstrahl (14) erzeugt ist und wobei die jeweiligen unterschiedlichen Energien in einem Bereich zwischen einer Niedrigenergie und einer Hochenergie liegen, die zu einer Energiedichte im Brennpunkt (54) unterhalb der Energiedichteschwelle für LIOB des Kalibrierelements (18) und zu einer Hochenergiedichte im Brennpunkt (54) oberhalb der Energiedichteschwelle für LIOB des Kalibrierelements (18) führen, um eine Energiedichte für den Brennpunkt des Laserstrahls (14) zu bestimmen, wobei vorzugsweise eine Vielzahl von Testmustern miteinander verglichen sind, um eine Einheitlichkeit der Energiedichte im Brennpunkt des Laserstrahls (14) zu bestimmen.

7. Verfahren zum Kalibrieren der Position eines Brennpunkts (54) eines chirurgischen Lasersystems (10), umfassend die Schritte:
Bereitstellen eines Mittels (12) zur Erzeugung eines Laserstrahls (14), wobei das Erzeugungsmittel (12) einen Basispunkt (16) definiert,
Befestigen eines Mittels (36) zum Kalibrieren des Laserstrahls (14) an dem Erzeugungsmittel (12), wobei das Kalibriermittel (36) eine gekrümmte Kalibrierelementoberfläche (46) mit einer vorbestimmten Krümmung aufweist, wobei die Oberfläche (46) eine im Wesentlichen senkrecht zu ihr verlaufende zentrale Achse (48) definiert und wobei die Oberfläche (46) in einem vorgegebenen Abstand von dem Basispunkt (16) des Erzeugungsmittels (12) angeordnet ist,
Erzeugen des Laserstrahls (14) mit dem Erzeugungsmittel (12), um den Laserstrahl (14) durch die Kalibrierelementoberfläche (46) zu leiten,
Fokussieren des Laserstrahls (14) in einen Brennpunkt (54) an einem vorgewählten anfänglichen Ort (56) in einem Abstand von der Kalibrierelementoberfläche (46),
Bewegen des Brennpunkts um einen Abstand "d", bis ein Laser Induced Optical Breakdown (LIOB) an einem endgültigen Ort (60) auf der Oberfläche (46) des Kalibrierelements (18) induziert ist,
Bestimmen des Abstands "d" und
Vergleichen des Abstands "d" mit einem vorbestimmten Wert, um das Lasersystem (10) zu kalibrieren.

8. Verfahren nach Anspruch 7, weiter umfassend den Schritt des Messens eines radialen Abstands "r" des endgültigen Orts (60) von der zentralen Achse (48), um das Lasersystem (10) zu kalibrieren.

9. Verfahren nach Anspruch 7, weiter umfassend die Schritte:
Lenken des Brennpunkts (54) weg von der Oberfläche (46) und
Wiederholen der Bewegungs- und Lenkungsschritte, um LIOB an einer Vielzahl von endgültigen Orten (60) auf der Oberfläche (46) zu iduzieren, um eine "Neigung" und eine "Dezentrierung" des Laserstrahls (14) zu kalibrieren.

10. Verfahren nach Anspruch 9, wobei die Vielzahl von endgültigen Orten (60) ein Testmuster erzeugt, und wobei die Bewegungs- und Lenkungsschritte mehrfach durchgeführt werden, um eine Vielzahl von Testmustern zu erzeugen, wobei jedes Testmuster jeweils unter Verwendung einer unterschiedlichen Energie in dem Laserstrahl (14) erzeugt wird, und wobei die jeweiligen unterschiedlichen Energien in einem Bereich zwischen einer Niedrigenergie und einer Hochenergie liegen, die zu einer Energiedichte im Brennpunkt (54) unterhalb der Energiedichteschwelle für LIOB des Kalibrierelements (18) und einer Hochenergiedichte im Brennpunkt (54) oberhalb der Energiedichteschwelle für LIOB des Kalibrierelements (18) führen, um eine Energiedichte für den Brennpunkt des Laserstrahls (14) zu bestimmen, wobei das Verfahren vorzugsweise weiterhin den Schritt des Vergleichens der Vielzahl von Testmustern miteinander umfasst, um eine Einheitlichkeit der Energiedichte in dem Brennpunkt des Laserstrahls (14) zu bestimmen.

## Revendications

1. Système de laser chirurgical (10) pouvant être étalonné, adapté pour étalonner la position d'un foyer (54) du système de laser (10), qui comprend :
une unité de laser (12) pour générer un faisceau laser (14), dans lequel ladite unité de laser (12) définit un plan de référence de base (16) ;
un corps d'étalonnage (36) monté sur ladite unité de laser (12) ;
un élément d'étalonnage (18) fixé audit corps d'étalonnage et comportant une surface (46) incurvée avec une courbure prédéterminée, dans lequel la surface (46) définit un axe central (48) sensiblement perpendiculaire à celle-ci, et dans lequel ledit élément d'étalonnage (18) est fixé audit corps d'étalonnage (36) pour positionner la surface (46) dudit élément d'étalonnage (18) à une distance prédéterminée du plan de référence de base (16) de ladite unité de laser (12) ;
des moyens optiques (22) pour focaliser le faisceau laser (14) sur un foyer (54) à un emplacement initial (56) sélectionné au préalable à distance de la surface (46) de l'élément d'étalonnage (18) ;
des moyens pour déplacer le foyer vers la surface (46) dudit élément d'étalonnage (18) sur une distance « d », jusqu'à ce qu'un claquage optique induit par laser (LIOB) soit induit à un emplacement final (60) sur la surface (46) dudit élément d'étalonnage (18) ;
des moyens pour déterminer la distance « d » et
des moyens pour comparer la distance « d » à une valeur prédéterminée pour étalonner le système de laser (10).

2. Système (10) selon la revendication 1, comprenant en outre des moyens pour mesurer une distance radiale « r » de l'emplacement final (60) par rapport à l'axe central (48) pour étalonner le système de laser (10).

3. Système (10) selon la revendication 1, dans lequel la surface (46) a un rayon de courbure dans une plage entre environ huit et douze millimètres.

4. Système (10) selon la revendication 1, dans lequel ledit élément d'étalonnage (18) est réalisé en un matériau ayant un seuil d'énergie prédéterminé pour le LIOB.

5. Système (10) selon la revendication 1, dans lequel une pluralité d'emplacements finaux (60) sont utilisés pour étalonner une « inclinaison » et un « décentrage » pour le système de laser (10), dans lequel, de préférence, une pluralité d'emplacements finaux (60) créent un motif de test.

6. Système (10) selon la revendication 5, dans lequel chaque motif de test est respectivement créé en utilisant une énergie différente dans le faisceau laser (14), et dans lequel les différentes énergies respectives sont dans une plage entre une faible énergie et une énergie élevée, produisant une densité d'énergie au foyer (54) inférieure au seuil de densité d'énergie pour le LIOB de l'élément d'étalonnage (18), et une densité d'énergie élevée au foyer (54) supérieure au seuil de densité d'énergie pour le LIOB dudit élément d'étalonnage (18) pour déterminer une densité d'énergie pour la tache focale du faisceau laser (14), dans lequel, de préférence, une pluralité de motifs de test sont comparés les uns aux autres pour déterminer une uniformité pour la densité d'énergie au niveau de la tache focale du faisceau laser (14).

7. Procédé pour étalonner la position d'un foyer (54) d'un système de laser chirurgical (10), qui comprend les étapes consistant à :
fournir des moyens (12) pour générer un faisceau laser (14), dans lequel lesdits moyens de génération (12) définissent un plan de référence de base (16) ;
monter des moyens (36) pour étalonner le faisceau laser (14) sur les moyens de génération (12), lesdits moyens d'étalonnage (36) comprenant une surface d'élément d'étalonnage (46) incurvée avec une courbure prédéterminée, dans lequel la surface (46) définit un axe central (48) sensiblement perpendiculaire à celle-ci, et dans lequel la surface (46) est positionnée à une distance prédéterminée du plan de référence de base (16) desdits moyens de génération (12) ;
générer le faisceau laser (14) par les moyens de génération (12) pour faire passer le faisceau laser (14) à travers ladite surface d'élément d'étalonnage (46) ;
focaliser le faisceau laser (14) sur un foyer (54) à un emplacement initial (56) sélectionné au préalable à distance de la surface d'élément d'étalonnage (46) ;
déplacer le foyer sur une distance "d", jusqu'à ce qu'un claquage optique induit par laser (LIOB) soit induit à un emplacement final (60) sur la surface (46) dudit élément d'étalonnage (18) ;
déterminer la distance « d » ; et
comparer la distance « d » à une valeur prédéterminée pour étalonner le système de laser (10).

8. Procédé selon la revendication 7, comprenant en outre l'étape consistant à mesurer une distance radiale « r » de l'emplacement final (60) par rapport à l'axe central (48) pour étalonner le système de laser (10).

9. Procédé selon la revendication 7, comprenant en outre les étapes consistant à :
diriger le foyer (54) loin de la surface (46) ; et
répéter les étapes de déplacement et d'orientation pour induire le LIOB à une pluralité d'emplacements finaux (60) sur la surface (46) afin d'étalonner une « inclinaison » et un « décentrage » du faisceau laser (14).

10. Procédé selon la revendication 9, dans lequel la pluralité d'emplacements finaux (60) créent un motif de test, et dans lequel les étapes de déplacement et d'orientation sont effectuées de multiples fois pour créer une pluralité de motifs de test, chaque motif de test étant respectivement créé en utilisant une énergie différente dans le faisceau laser (14), et dans lequel les différentes énergies respectives sont dans une plage entre une faible énergie et une énergie élevée, produisant une densité d'énergie au foyer (54) inférieure au seuil de densité d'énergie pour le LIOB de l'élément d'étalonnage (18), et une densité d'énergie élevée au foyer (54) supérieure au seuil de densité d'énergie pour le LIOB dudit élément d'étalonnage (18) pour déterminer une densité d'énergie pour la tache focale du faisceau laser (14), comprenant en outre, de préférence, l'étape de comparaison de la pluralité de motifs de test les uns aux autres pour déterminer une uniformité pour la densité d'énergie au niveau de la tache focale du faisceau laser (14).
